# EUROPEAN PATENT APPLICATION

(11) **EP 4 278 975 A1**
(43) Date of publication of application: **22.11.2023**
(21) Application number: 22173814.9
(22) Date of filing: 17.05.2022
(51) Int. Cl.: A61B 6/03, A61B 6/00, G01T 1/29, A61B 6/06, A61B 6/08

(54) **IMAGING SYSTEM FOR DETECTING X-RAY AND GAMMA RADIATION**

(71) Applicant: Koninklijke Philips N.V., 5656 AG Eindhoven (NL)
(72) Inventor: WIECZOREK, Herfried Karl, Eindhoven (NL)
(74) Representative: Philips Intellectual Property & Standards

(57) **Abstract**

An imaging system (100) includes a gantry (110), an X-ray source (120), and a detector module (130) configured to detect both X-ray radiation emitted by the X-ray source (120), and gamma radiation. The detector module (130) is movable with respect to the gantry (110) for adjusting a field of view of the detector module with respect to the axis of rotation.

## Description

### TECHNICAL FIELD

The present disclosure relates to an imaging system for detecting X-ray and gamma radiation. An imaging system, an imaging method, and a computer program product, are disclosed.

### BACKGROUND

Various clinical investigations make use of gamma data representing a distribution of a radiotracer distribution in a region of interest in a subject, as well as X-ray attenuation data for the region of interest. For instance, gamma data is often acquired in order to generate a single photon emission computed tomography "SPECT" image, or a scintigraphy image for the region of interest. Such images are used to investigate the physiological behavior of biological matter within the region of interest. X-ray attenuation data is often also acquired for the region of interest and used to generate a so-called "attenuation map" in order to account for scattering and attenuation of the gamma photons emitted by the radiotracer. Gamma photon scattering arises from interactions between the gamma photons and the biological matter in the region of interest. By accounting for scattering, the quality of the resulting SPECT or scintigraphy image can be improved.

Gamma data representing a distribution of a radiotracer distribution in the region of interest, may in general be generated by a gamma detector. The radiotracer is injected into the blood stream, and subsequently accumulates, or is "uptaken" in the region of interest in response to physiological activity. The gamma detector that is used to determine the distribution of the radiotracer is also known as a gamma camera, and may form part of a SPECT imaging system or a scintigraphy imaging system. Gamma data thus provides physiological, or "functional" information relating to the region of interest. X-ray attenuation data may in general be generated by an X-ray imaging system, or a computed tomography "CT" imaging system. X-ray attenuation data provides structural information relating to the anatomy in a region of interest.

Often, separate imaging systems are used to acquire the gamma data the X-ray attenuation data. Sometimes, the imaging systems that are used to generate the two sets of data are disposed in different locations. The two sets of data may even be acquired on different days. This leads to challenges when accounting for scatter in the resulting SPECT or scintigraphy images because the region of interest may deform between acquisition of the two data sets. This situation can be improved by concatenating the two separate imaging systems, and translating the subject between them in order to acquire the two data sets. With such an arrangement, the two data sets can be acquired sequentially, and also within a short time interval of each other. This reduces the amount of deformation of the region of interest between the resulting images.

Attempts have been made to further reduce the amount of deformation of the region of interest that occurs between acquisition of the two data sets by providing an imaging system that can generate both X-ray attenuation data, and gamma data. Such a combined imaging system has the advantage that the subject does not necessarily need to be moved between acquiring the two data sets. For instance, a document US 2005/0207526 A1 discloses an imaging system that includes a gantry unit having an X-ray source for generating X-rays and a CZT detector configured to detect emission gamma photons and transmission X-ray photons, the gantry moving the X-ray source and detector along an image acquisition path between at least first and second imaging positions.

However, there remains a need to provide an improved imaging system that is capable of generating both X-ray attenuation data, and gamma data.

### SUMMARY

According to one aspect of the present disclosure, an imaging system is provided. The imaging system includes: a gantry, an X-ray source, and a detector module configured to detect both X-ray radiation emitted by the X-ray source, and gamma radiation. The X-ray source and the detector module are coupled to the gantry in an opposing configuration for generating both X-ray attenuation data representing an attenuation of the X-ray radiation passing through an imaging region disposed between the X-ray source and the detector module, and gamma data representing gamma radiation received from the imaging region. The gantry comprises an axis of rotation, and the gantry is configured to rotate around the axis of rotation for detecting the X-ray radiation and the gamma radiation with the detector module from a plurality of angular positions around the axis of rotation. The detector module is movable with respect to the gantry for adjusting a field of view of the detector module with respect to the axis of rotation.

Since the detector module is movable with respect to the gantry, the field of view of the detector module can be adjusted with respect to the axis of rotation. This provides control over the imaging region from which data is acquired. For instance, gamma data for a 2D scintigraphy image is typically acquired with the detector module static and in a single angular position around the axis of rotation. By providing the ability to move the detector module with respect to the gantry, the position of the field of view for gamma radiation, and thus the imaging region for gamma radiation, may be adjusted in order to provide gamma data for a 2D scintigraphy image of a desired region of interest. By way of another example, gamma data for a SPECT image, i.e. a volumetric image, may be acquired from multiple angular positions around the axis of rotation by rotating the detector module around the axis of rotation. The detector module may be rotated continuously, or in a stepped manner, around the axis of rotation, and data may be acquired by the detector module whilst the detector module rotates around the axis of rotation, or with the detector module paused in each of multiple angular positions q defined by the stepping, around the axis of rotation. By providing the ability to move the detector module with respect to the gantry, the field of view of the detector module can be adjusted with respect to the axis of rotation, and this in-turn controls the shape of the imaging region for gamma radiation. This is because when the detector module is rotated around the axis of rotation to acquire gamma data from multiple angular positions around the axis of rotation, the shape of the imaging region for gamma radiation is determined by the volume swept by the field of view of the detector module during the rotation. Thus by providing that the detector module is movable with respect to the gantry, the shape of the imaging region can be controlled in order to provide gamma data for a SPECT image of a desired region of interest.

Similar advantages arise from making the detector module movable with respect to the gantry when X-ray attenuation data is acquired. Corresponding advantages exist in the case that X-ray attenuation data is acquired with the detector module static and in a single angular position around the axis of rotation, i.e. when acquiring X-ray attenuation data for an X-ray projection image, as well as in the case that X-ray attenuation data is acquired from multiple angular positions around the axis of rotation by rotating the detector module around the axis of rotation, i.e. in order to acquire X-ray attenuation data for a CT image.

Thus, by providing control over the imaging region from which data is acquired, the flexibility of the imaging system 100 is improved.

A further advantage of providing a detector module that can detect both X-ray radiation as well as gamma radiation in a common imaging system is the potential to acquire gamma data and X-ray attenuation data with improved correspondence between the two data sets. This is because the gamma data and X-ray attenuation data may be acquired without the need to move the subject between acquisition of the data sets.

According to another aspect of the present disclosure, the detector module includes a first detector array configured to detect the X-ray radiation, and a second detector array configured to detect the gamma radiation. The second detector array comprises a length dimension and a width dimension, the length dimension being orthogonal to the width dimension and the length dimension exceeding the width dimension. The detector module is rotatably movable with respect to the gantry for adjusting the field of view of the detector module with respect to the axis of rotation. The detector module is rotatably movable with respect to the gantry between a first orientation wherein the length dimension of the second detector array extends along the axis of rotation, and a second orientation wherein the length dimension of the second detector array extends transversely with respect to the axis of rotation.

The use of a detector module in which the second detector array has such length and width dimensions, or such an "aspect ratio", defined by the ratio of its length to its width, permits the weight of the detector module to be reduced. The ability to rotate the detector module in accordance with this aspect permits the acquisition of gamma data for 2D scintigraphy images, and likewise X-ray attenuation data for X-ray projection images, with an aspect ratio that is appropriate for the imaged region of interest. For instance, data for the heart may be acquired with the length dimension of the second detector array extending transversely with respect to the axis of rotation, i.e. with the detector module positioned in the second orientation. By contrast, for lung imaging, or for kidney imaging, the detector module may be positioned in the first orientation, i.e. with the length dimension of the second detector array extending along the axis of rotation. Similarly, when X-ray attenuation data and/or gamma data is acquired from multiple angular positions around the axis of rotation by rotating the detector module around the axis of rotation, the shape of the imaging region from which data is acquired can be controlled by positioning the detector module in the first orientation, or in the second orientation. By providing the imaging region with a shape that is appropriate for the region of interest, truncation artifacts in SPECT images that are reconstructed from the gamma data may be reduced. Such truncation artifacts result from detected gamma radiation that is emitted from regions that are outside the imaging region, or from regions that are not sampled over a complete range of angular positions around the axis of rotation.

According to another aspect of the present disclosure, the detector module comprises a first detector array configured to detect the X-ray radiation, and a second detector array configured to detect the gamma radiation. The detector module is translatably movable with respect to the gantry for adjusting the field of view of the detector module with respect to the axis of rotation. The detector module is translatably movable with respect to the gantry in a transaxial direction with respect to the axis of rotation between a first transaxial position wherein a line extending normally from the midpoint of the second detector array lies on one side of the axis of rotation, and a second transaxial position wherein the line extending normally from the midpoint of the second detector array lies on the opposite side of the axis of rotation.

The ability to translate the detector module in accordance with this aspect permits the acquisition of gamma data, and likewise X-ray attenuation data, from an imaging region that is appropriate for the imaged region of interest. For instance, when data is acquired with the detector module static and in a single angular position around the axis of rotation, the data may be acquired from imaging regions that have different offsets with respect to the axis of rotation. This may be beneficial for generating data for organs such as the heart, and individual lungs, and which are offset with respect to the axis of rotation. Moreover, when gamma data for a SPECT image is acquired by rotating the detector module around the axis of rotation, the ability to move the detector module between the first transaxial position and the second transaxial position permits the acquisition of a complete set of gamma data for an imaging region that is extended transaxially as compared to the imaging region provided by a detector module in which a line extending normally from the midpoint of the second detector array passes through the axis of rotation. As compared to a detector module in which a line extending normally from the midpoint of the second detector array passes through the axis of rotation, a detector module that is offset such that a line extending normally from the midpoint of the second detector array lies on one side of the axis of rotation, permits image data to be acquired from a swept volume, i.e. an imaging region, that is transaxially-extended, i.e. extended in a transaxial direction with respect to the axis of rotation. This principle is employed in so-called "offset geometry" cone beam CT imaging systems. However, the acquisition of gamma data for a SPECT image by rotating a detector that is disposed in such a transaxially-offset position, differs from the acquisition of X-ray attenuation data for a CT image. X-ray attenuation data is indifferent to the direction in which the X-ray radiation passes through a region of interest. This permits a full set of X-ray attenuation data to be acquired for a CT image by rotating a transaxially-offset X-ray detector through a single revolution around its axis of rotation. By contrast, the direction from which gamma radiation is received is relevant to the reconstruction of gamma data into a SPECT image. Consequently, in order to acquire a full set of gamma data for a SPECT image using a transaxially-offset detector, two rotations around the region of interest are necessary: one with the gamma detector in first transaxial position wherein a line extending normally from the midpoint of the second detector array lies on one side of the axis of rotation, and one with the gamma detector in a second transaxial position wherein the line extending normally from the midpoint of the second detector array lies on the opposite side of the axis of rotation. Thus, a full set of gamma data for a SPECT image with a transaxially-extended imaging region may be acquired by providing that the detector module is translatably movable between the first transaxial position and the second transaxial position.

Further aspects, features, and advantages of the present disclosure will become apparent from the following description of examples, which is made with reference to the accompanying drawings.

### BRIEF DESCRIPTION OF THE DRAWINGS

Fig. 1 is a schematic diagram illustrating a first example of an imaging system 100 including a detector module 130 that is coupled to a gantry 110 and wherein the detector module is movable with respect to the gantry, in accordance with some aspects of the present disclosure.
Fig. 2 is a schematic diagram illustrating a second example of an imaging system 100 including a detector module 130 that is coupled to a gantry 110 and wherein the detector module is movable with respect to the gantry, in accordance with some aspects of the present disclosure.
Fig. 3 is a schematic diagram illustrating a third example of an imaging system 100 including a detector module 130 that is coupled to a gantry 110 and wherein the detector module is movable with respect to the gantry, in accordance with some aspects of the present disclosure.
Fig. 4 is a schematic diagram illustrating a fourth example of an imaging system 100 including a detector module 130 that is coupled to a gantry 110 and wherein the detector module is movable with respect to the gantry, in accordance with some aspects of the present disclosure.
Fig. 5 is a schematic diagram illustrating a fifth example of an imaging system 100 including a detector module 130 that is coupled to a gantry 110 and wherein the detector module is movable with respect to the gantry, in accordance with some aspects of the present disclosure.
Fig. 6 is a schematic diagram illustrating an example of imaging system 100 including a processor 260, in accordance with some aspects of the present disclosure.
Fig. 7 is a flowchart illustrating an example of an imaging method, in accordance with some aspects of the present disclosure.

### DETAILED DESCRIPTION

Examples of the present disclosure are provided with reference to the following description and figures. In this description, for the purposes of explanation, numerous specific details of certain examples are set forth. Reference in the specification to "an example", "an implementation" or similar language means that a feature, structure, or characteristic described in connection with the example is included in at least that one example. It is also to be appreciated that features described in relation to one example may also be used in another example, and that all features are not necessarily duplicated in each example for the sake of brevity. For instance, operations described in relation to a system, may be implemented in a computer program product in a corresponding manner.

In the following description, reference is made to examples of an imaging system that includes a gantry in the form of a C-arm. The C-arm gantry supports an X-ray source and a detector module. Many current C-arm based imaging systems include an X-ray source and an X-ray detector for acquiring X-ray attenuation data in order to generate projection images, i.e. 2D images. However, it is noted that the imaging system disclosed herein is not limited to use in acquiring data for projection images. Nor is the imaging system disclosed herein limited to use with a C-shaped gantry. The data acquired from the imaging system disclosed herein may be used to generate, or more specifically, reconstruct, volumetric images such as CT images and SPECT images, as well as to generate 2D or "projection" images such as X-ray projection images and gamma scintigraphy images. Moreover, the gantry may have a different shape to the example C-shape illustrated herein. For example, the gantry may alternatively have an oval shape, a rectangular shape, or a circular shape. By way of another example, the gantry may be provided by a robotic arm. It is also noted that the X-ray source and the detector module disclosed herein may alternatively be coupled to the gantry of a CT imaging system. In other words, the imaging system may be a CT imaging system.

In the following description, reference is also made to imaging systems that are used to acquire data for imaging various regions of interest in a subject. For instance, in some examples, reference is made to imaging systems in which data is acquired for imaging the heart. However, it is to be appreciated that these examples are used purely for illustration purposes, and that the imaging systems disclosed herein are not limited to use in acquiring data for a imaging particular region of interest. Thus, in general, the imaging systems may be used to acquire data for imaging any portion of the anatomy.

It is noted that various aspects of the methods disclosed herein may be implemented by a computer. Thus, the methods may be provided as a non-transitory computer-readable storage medium including computer-readable instructions stored thereon, which, when executed by at least one processor, cause the at least one processor to perform the method. In other words, the computer-implemented methods may be implemented in a computer program product. The computer program product can be provided by dedicated hardware, or hardware capable of running the software. When provided by a processor, the functions of the method features can be provided by a single dedicated processor, or by a single shared processor, or by a plurality of individual processors, some of which can be shared. The functions of one or more of the method features may for instance be provided by processors that are shared within a networked processing architecture such as a client/server architecture, a peer-to-peer architecture, the Internet, or the Cloud.

The explicit use of the terms "processor" or "controller" should not be interpreted as exclusively referring to hardware capable of running software, and can implicitly include, but is not limited to, digital signal processor "DSP" hardware, read only memory "ROM" for storing software, random access memory "RAM", a non-volatile storage device, and the like. Furthermore, examples of the present disclosure can take the form of a computer program product accessible from a computer-usable storage medium, or a computer-readable storage medium, the computer program product providing program code for use by or in connection with a computer or any instruction execution system. For the purposes of this description, a computer-usable storage medium or a computer readable storage medium can be any apparatus that can comprise, store, communicate, propagate, or transport a program for use by or in connection with an instruction execution system, apparatus, or device. The medium can be an electronic, magnetic, optical, electromagnetic, infrared, or a semiconductor system or device or propagation medium. Examples of computer-readable media include semiconductor or solid state memories, magnetic tape, removable computer disks, random access memory "RAM", read-only memory "ROM", rigid magnetic disks and optical disks. Current examples of optical disks include compact disk-read only memory "CD-ROM", compact disk-read/write "CD-R/W", Blu-Ray^{™} and DVD.

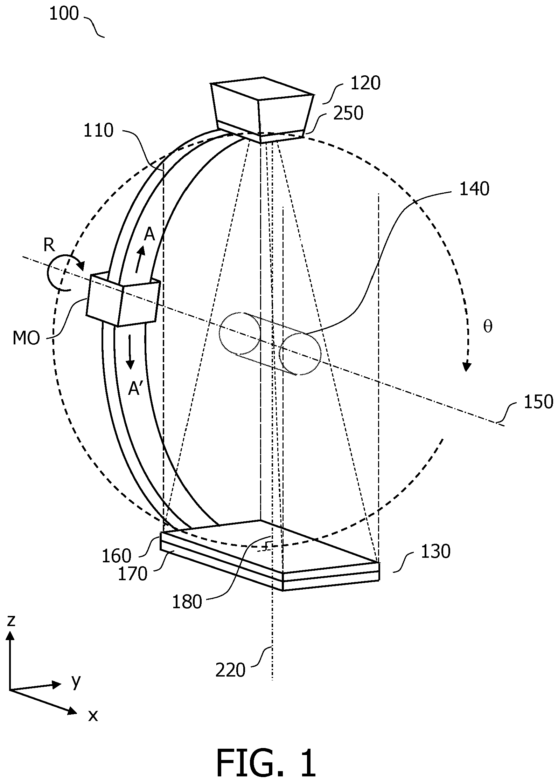

As mentioned above, there remains a need to provide an improved imaging system that is capable of generating both X-ray attenuation data, and gamma data. Thereto, an imaging system 100 is provided. Fig. 1 is a schematic diagram illustrating a first example of an imaging system 100 including a detector module 130 that is coupled to a gantry 110 and wherein the detector module is movable with respect to the gantry, in accordance with some aspects of the present disclosure.

With reference to Fig. 1, the imaging system 100 includes a gantry 110, an X-ray source 120, and a detector module 130 configured to detect both X-ray radiation emitted by the X-ray source 120, and gamma radiation. The X-ray source 120 and the detector module 130 are coupled to the gantry 110 in an opposing configuration for generating both X-ray attenuation data representing an attenuation of the X-ray radiation passing through an imaging region 140 disposed between the X-ray source and the detector module, and gamma data representing gamma radiation received from the imaging region 140. The gantry 110 comprises an axis of rotation 150, and the gantry is configured to rotate around the axis of rotation for detecting the X-ray radiation and the gamma radiation with the detector module from a plurality of angular positions q around the axis of rotation. The detector module 130 is movable with respect to the gantry 110 for adjusting a field of view of the detector module with respect to the axis of rotation.

In the example imaging system 100 illustrated in Fig. 1, the X-ray source 120 and the detector module 130 are coupled to the C-arm gantry 110. The X-ray source 120 and the detector module 130 are coupled to the C-arm gantry 110 at opposite positions in order to generate X-ray attenuation data representing an attenuation of the X-ray radiation passing through the imaging region 140 disposed between the X-ray source and the detector module.

In the example illustrated in Fig. 1, the detector module 130 includes a first detector array 160 configured to detect X-ray radiation, and a second detector array 170 configured to detect gamma radiation. A field of view of the detector module 130 for X-ray radiation is defined by the volumetric overlap between the X-ray radiation emitted by the X-ray source 120, and the detection area of the first detector array 160 that detects the X-ray radiation. In Fig. 1, this field of view is illustrated by way of the outermost short-dashed lines connecting the X-ray source 120 to the detector module 130.

The detector module 130 illustrated in Fig. 1 also has a field of view for gamma radiation. The field of view for gamma radiation is determined by the detection area of the second detector array 170 that detects gamma radiation. The field of view for gamma radiation may also be determined by a gamma radiation collimator and/or an anti-scatter grid that may be included in the detector module 130. In general, the field of view for gamma radiation extends outwards from the gamma radiation receiving face of the second detector array 180, and overlaps with the field of view for X-ray radiation. In one example, the field of view for gamma radiation extends convergently outwards from the gamma radiation receiving face of the second detector array 180 and overlaps with the field of view for X-ray radiation. The field of view for gamma radiation may have the shape of a four-sided pyramid with its apex disposed above the detector module 130 and wherein the edges of the pyramid that connect to the apex pass through the corners of the detector module. The field of view for gamma radiation may overlap exactly with the field of view for gamma radiation. For instance, the field of view for gamma radiation may correspond to the outermost short-dashed lines connecting the X-ray source 120 to the detector module 130 in Fig. 1. In this case, exact correspondence is provided between the fields of view for gamma and X-ray radiation. Another example of the field of view for gamma radiation is illustrated in Fig. 1 via the long-dashed lines that extend vertically upwards from the detector module 130. In this example, the field of view for gamma radiation extends normally outwards from the gamma radiation receiving face of the second detector array 180. Such fields of view for the gamma radiation may be defined by the septa of a SPECT collimator disposed on a radiation-receiving side of the second detector array 170.

In the example detector module 130 illustrated in Fig. 1, the first detector array 160 and the second detector array 170 comprise planar layers. The planar layers are stacked along a path 180 extending through both the X-ray source 120 and a center of the detector module 130, and the first detector array is disposed relatively closer to the X-ray source 120 than the second detector array 170. In this configuration, quanta from the relatively-lower energy X-ray radiation are detected by the first detector array 160, and quanta from the relatively-higher energy gamma radiation pass through the first detector array 160 and are detected by the second detector array 170. The use of this stacked configuration provides correspondence between the spatial distribution of detected X-ray radiation and the spatial distribution of detected gamma radiation.

The detector module 130 in this example may be used to acquire X-ray attenuation data and gamma data during time intervals that overlap one another. This provides exact correspondence between the spatial distribution of detected X-ray radiation and the spatial distribution of detected gamma radiation. It is noted that the X-ray attenuation data may in general be acquired over a shorter time interval than the gamma data. For example, depending on the X-ray tube current and voltage, an X-ray projection image may be acquired over a time interval of a few tens of milliseconds. By contrast, a gamma scintigraphy image may be acquired over a total time interval of 10 minutes to 20 minutes. A CT image acquired during a continuous single revolution of the gantry may be acquired over a total time interval of approximately 200 milliseconds to 300 milliseconds. A SPECT image may be acquired over a total time interval of approximately 30 minutes. Thus, when the X-ray attenuation data and the gamma data are acquired during time intervals that overlap one another, both types of data are acquired simultaneously during at least part of the time intervals over which the two types of data are acquired. The X-ray attenuation data that is acquired from the example detector module 130 may subsequently be used to generate an X-ray projection image, or a CT image. The X-ray projection image, or the CT image, may be used during the generation of the scintigraphy image, or the SPECT image, from the gamma data, to provide a scatter- and attenuation-corrected scintigraphy image, or a scatter- and attenuation-corrected SPECT image, respectively. Due to the improved correspondence between the spatial distribution of detected X-ray radiation and the spatial distribution of detected gamma radiation, an improved scatter- and attenuation-corrected scintigraphy image, or scatter- and attenuation-corrected SPECT image is provided.

The use of various X-ray sources as the X-ray source 120, and likewise various detectors in the detector module 130 illustrated in Fig. 1, is contemplated.

In some examples, the X-ray source 120 and the first detector array 160 that respectively generate and detect the X-ray radiation, are configured to generate X-ray attenuation data that represents X-ray attenuation within a single X-ray energy interval. In other examples, the X-ray source 120 and the first detector array 160 are configured to generate X-ray attenuation data that represents X-ray attenuation at a plurality of different energy intervals, i.e. spectral X-ray attenuation data. Thus, in general the X-ray source 120 may include one or more monochromatic sources, or one or more polychromatic sources, or a polychromatic source operated at different anode voltages, and the first detector array 160 may include: a detector for detecting a single X-ray energy interval, or a common detector for detecting multiple different X-ray energy intervals, or multiple detectors wherein each detector detects a different X-ray energy interval, or a multi-layer detector in which X-rays having energies within different X-ray energy intervals are detected by corresponding layers, or a so-called photon counting detector that bins detected X-ray photons into one of multiple energy intervals based on their individual energies.

The example X-ray source 120 illustrated in Fig. 1 also includes an X-ray collimator 250. The X-ray collimator 250 defines the lateral extent of X-ray radiation emitted by the X-ray source. The X-ray collimator 250 may include a plurality of movable plates that define the lateral extent of X-ray radiation emitted by the X-ray source. The movable plates absorb X-ray radiation. The movable plates may be formed from lead, for example. The movable plates can be moved so as to adjust the lateral extent of X-ray radiation emitted by the X-ray source. Thus, the movable plates may be adjusted in order to provide a beam of X-ray radiation with a desired shape. For instance, the movable plates may be used to limit a lateral extent of the X-ray radiation such that it overlaps with the detector module 130.

In the example detector module 130 illustrated in Fig. 1, the first detector array 160 includes an array of detector elements that detect a spatial distribution of received X-ray radiation. The detector elements may employ a so-called "indirect conversion" technique wherein X-ray quanta are detected by means of the optical pulses that are generated when the X-ray quanta are received by a scintillator material. Alternatively, the detector elements of the first detector array 160 may employ a so-called "direct conversion" technique. Detector elements that employ an indirect conversion technique include a layer of scintillator material that converts the received X-ray quanta into optical pulses, and an associated photodetector that converts the optical pulses onto electrical signals. Scintillator materials such as thallium doped caesium iodide, i.e. CsI(Tl), are often used for this purpose. The associated photodetectors may be provided by solid state photodiodes, or by avalanche photodiodes, silicon photomultiplier "SiPM"s, and so forth. The associated photodetectors may be operated in a so-called integrating mode, and wherein the optical photons in an optical pulse generated by a single X-ray quant are integrated over time in order to determine the energy of the received X-ray quant. In some cases, the associated photodetector may be operated in a so-called "photon counting" mode, and wherein a count of the number of individual optical photons in an optical pulse is generated. For example, solid state detectors such as SiPM detectors may be configured as "photon counting" detectors. The ability to count individual photons in the optical pulse provides accurate information on the energy of the received X-ray quanta. This information may be used to distinguish individual X-ray quanta that have been subjected to scattering from non-scattered X-ray quanta. Detector elements that employ direct conversion techniques, may be formed from materials such as cadmium telluride, i.e. CdTe, and cadmium zinc telluride "CZT", or perovskite layers like methyl amine lead iodide "MAPI" or similar compounds, and which directly convert received X-ray quanta into an electrical signal.

In the example detector module 130 illustrated in Fig. 1, the second detector array 170 includes an array of detector elements that detect a spatial distribution of received gamma radiation. The detector elements may employ an indirect conversion technique wherein gamma quanta are detected by means of the optical pulses that are generated when the gamma quanta are received by a scintillator material. Alternatively, the detector elements of the second detector array 170 may employ a direct conversion technique. Detector elements that employ an indirect conversion technique include a layer of scintillator material that converts the received gamma quanta into optical pulses, and an associated photodetector that converts the optical pulses onto electrical signals. Scintillator materials such as thallium doped sodium iodide, i.e. NaI(Tl) are often used for this purpose. The associated photodetectors may be provided by photomultiplier tube "PMT" detectors, or by solid state photodiodes, avalanche photodiodes, silicon photomultiplier "SiPM"s, and so forth. The associated photodetectors may be operated in an integrating mode, and wherein the optical photons in an optical pulse generated by a single gamma quant are integrated over time in order to determine the energy of the received gamma quant. In some cases, the associated photodetector may be operated in a photon counting mode, and wherein a count of the number of individual optical photons in an optical pulse is generated. For example, solid state detectors such as SiPM detectors may be configured as "photon counting" detectors. The ability to count individual photons in the optical pulse provides accurate information on the energy of the received gamma quanta. This information may be used to distinguish individual gamma quanta that have been subjected to scattering from non-scattered gamma quanta. Detector elements that employ so-called direct conversion techniques, may be formed from materials such as cadmium zinc telluride "CZT" that directly convert received gamma quanta into an electrical signal.

Thus, the example detector module 130 illustrated in Fig. 1 is configured to generate both X-ray attenuation data representing an attenuation of the X-ray radiation passing through an imaging region 140 disposed between the X-ray source and the detector module, as well as gamma data representing gamma radiation received from the imaging region 140.

The gamma radiation that is detected by the detector module 130 may be emitted by a radiotracer that is distributed within the region of interest. A radiotracer such as Technetium-99m, i.e. ^{99m}Tc, is injected into the blood stream of a subject for this purpose. Technetium-99m is often used to provide gamma data for generating SPECT images, or scintigraphy images. The gamma quanta emitted by Technetium-99m have an energy of approximately 140 keV. Other radiotracers that are currently used to generate SPECT images, or scintigraphy images include Indium-111, which emits gamma quanta with an energy of approximately 245 keV, and Iodine-131, which emits gamma quanta with an energy of approximately 364 keV The radiotracers that are used to generate SPECT images, or scintigraphy images, typically emit gamma quanta that have relatively lower energies than the gamma quanta that are used to generate position emission tomography "PET" images. In PET imaging, the radiotracer fluorine-18-fluorodeoxyglucose "FDG" is often used, and this generates gamma quanta with an energy of approximately 511 keV. In some examples, the second detector array 170 may be configured to detect gamma quanta having an energy of less than approximately 400 keV. This facilitates the detection of gamma quanta from radiotracers that are typically used for generating SPECT images, or scintigraphy images.

With continued reference to Fig. 1, the gantry 110 includes an axis of rotation 150. The gantry 110 is configured to rotate around the axis of rotation in order to detect the X-ray radiation and the gamma radiation with the detector module from a plurality of angular positions q around the axis of rotation. In this regard, the gantry may be supported by a mounting MO that includes an axle and/or a bearing, and one or more actuators such as a motor, and so forth, that rotate the mounting MO as indicated by the arrow R. The rotation of the mounting MO has the effect of rotating the gantry 110 around the axis of rotation through the plurality of angular positions q. The example gantry 110 illustrated in Fig. 1 is configured to rotate in a so-called "propellor mode". During an imaging procedure, a subject may be arranged such that the longitudinal axis of the subject extends generally along the axis of rotation 150. This configuration may be used to image a subject's head, for example. It is noted that in the example illustrated in Fig. 1, the gantry 110 may also be provided with additional degrees of freedom. For example, the gantry may be movable with respect to the axis of rotation as indicated by the arrows A, A' in order to provide a desired perspective of a region of interest in the subject.

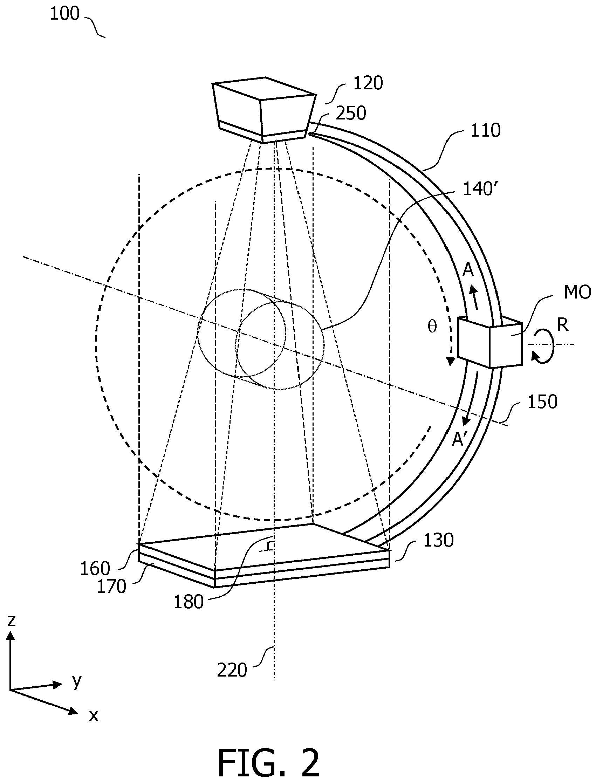

In an alternative configuration, the gantry 110 illustrated in Fig. 1 may be rotated about the z-axis, and positioned such that the gantry lies in the y-z plane. In this configuration, the gantry 110 may be configured to rotate around the axis of rotation 150 by moving the gantry 110 with respect to the mounting MO and in the direction of the arrows A, A'. This example is illustrated in Fig. 2, which is a schematic diagram illustrating a second example of an imaging system 100 including a detector module 130 that is coupled to a gantry 110 and wherein the detector module is movable with respect to the gantry, in accordance with some aspects of the present disclosure. In the configuration illustrated in Fig. 2, the one or more actuators described above with reference to Fig. 1 are configured to move the gantry with respect to the mounting MO as indicated by the arrows A, A'. The mounting may for example be provided with a rack and pinion arrangement that facilitate this movement. The effect of the movement of the gantry with respect to the mounting MO is that the gantry is rotated around the axis of rotation 150 through the plurality of angular positions q. The configuration illustrated in Fig. 2, therefore provides rotation of the gantry 110 around the same axis of rotation 150 as illustrated in Fig. 1. During an imaging procedure, a subject may be positioned such that the longitudinal axis of the subject extends generally along the axis of rotation 150. As compared to the example illustrated in Fig. 1, the example illustrated in Fig. 2 provides improved access to regions of interest in the subject that are located mid-way along the subject's axis. It is noted that in the configuration illustrated in Fig. 2, the one or more actuators may be used to rotate the mounting MO as indicated by the arrow R, in order to provide a desired perspective of a region of interest in the subject.

In some examples, the gantry 110 described above with reference to Fig. 1 and Fig. 1 may be rotated around the axis of rotation 150, and data may be acquired by the detector module 130 with the detector module static and in a single angular position q around the axis of rotation. For example, X-ray attenuation data for generating an X-ray projection image, or gamma data for generating a scintigraphy image, may be acquired with the detector module static and in a single angular position q around the axis of rotation. In other examples, the gantry 110 may be rotated continuously, or in a stepped manner, around the axis of rotation, and data may be acquired by the detector module whilst the detector module rotates around the axis of rotation, or with the detector module paused in each of multiple angular positions q defined by the stepping, around the axis of rotation.

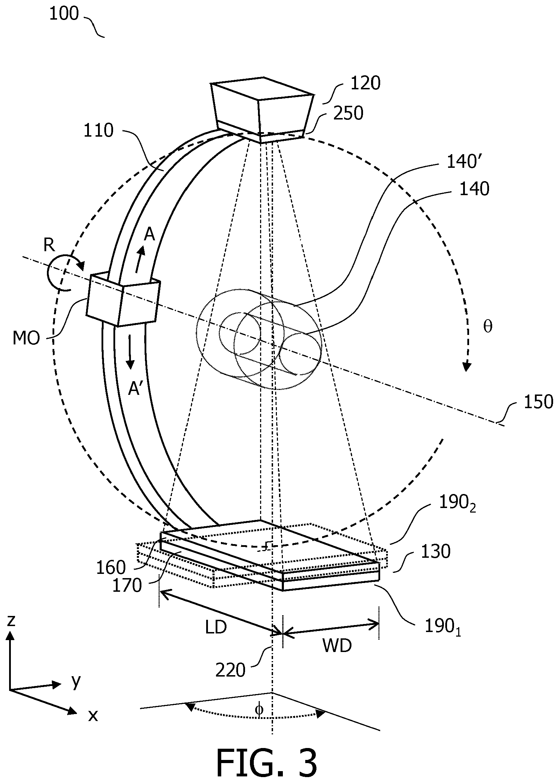

With continued reference to Fig. 1 and Fig. 2, the detector module 130 is movable with respect to the gantry 110 for adjusting a field of view of the detector module with respect to the axis of rotation. In this regard, the detector module 130 may be movable with various degrees of freedom, some of which are described below with reference to Fig. 3 - Fig. 5.

In general, since the detector module 130 is movable with respect to the gantry 110, the field of view of the detector module can be adjusted with respect to the axis of rotation. This provides control over the imaging region from which data is acquired. For instance, gamma data for a 2D scintigraphy image is typically acquired with the detector module static and in a single angular position around the axis of rotation. By providing the ability to move the detector module 130 with respect to the gantry 110, the position of the field of view for gamma radiation, and thus the imaging region for gamma radiation, may be adjusted in order to provide gamma data for a 2D scintigraphy image of a desired region of interest. By way of another example, gamma data for a SPECT image, i.e. a volumetric image, may be acquired from multiple angular positions around the axis of rotation by rotating the detector module 130 around the axis of rotation 150. The detector module 130 may be rotated continuously, or in a stepped manner, around the axis of rotation, and data may be acquired by the detector module whilst the detector module rotates around the axis of rotation, or with the detector module paused in each of multiple angular positions q defined by the stepping, around the axis of rotation. By providing the ability to move the detector module 130 with respect to the gantry 110, the field of view of the detector module can be adjusted with respect to the axis of rotation, and this in-turn controls the shape of the imaging region for gamma radiation. This is because when the detector module is rotated around the axis of rotation to acquire gamma data from multiple angular positions around the axis of rotation, the shape of the imaging region for gamma radiation is determined by the volume swept by the field of view of the detector module 130 during the rotation. Thus by providing that the detector module is movable with respect to the gantry, the shape of the imaging region, or more specifically by the volume swept by the field of view of the second detector array 170 that detects gamma radiation, can be controlled in order to provide gamma data for a SPECT image of a desired region of interest.

Similar advantages arise from making the detector module 130 movable with respect to the gantry when X-ray attenuation data is acquired. Corresponding advantages exist in the case that X-ray attenuation data is acquired with the detector module 130 static and in a single angular position around the axis of rotation, i.e. when acquiring X-ray attenuation data for an X-ray projection image, as well as in the case that X-ray attenuation data is acquired from multiple angular positions around the axis of rotation by rotating the detector module around the axis of rotation, i.e. in order to acquire X-ray attenuation data for a CT image. By way of an example, the imaging region 140 for X-ray attenuation data is illustrated in Fig. 1. The imaging region for X-ray attenuation data is determined by the volume swept by the field of view of the detector module 130, or more specifically the volume swept by the field of view of the first detector array 160 during the rotation. In the illustrated example, the imaging region 140 has a cylindrical shape by virtue of the rotation, which in this example is a complete revolution. The imaging region for gamma data is determined in a similar manner, i.e. by the volume swept by the field of view of the second detector array 170 during the rotation. The imaging region for gamma data overlaps with the imaging region or X-ray data.

Thus, by providing control over the imaging region from which data is acquired, the flexibility of the imaging system 100 is improved.

Fig. 3 is a schematic diagram illustrating a third example of an imaging system 100 including a detector module 130 that is coupled to a gantry 110 and wherein the detector module is movable with respect to the gantry, in accordance with some aspects of the present disclosure. Fig. 3 includes some of the features described above with reference to Fig. 1. It is noted that features illustrated in Fig. 3 that are also illustrated in Fig. 1 provide corresponding functionality, and that their functionality is not duplicated here for the sake of brevity. With reference to Fig. 3, the second detector array 170 comprises a length dimension LD and a width dimension WD. The length dimension is measured orthogonally with respect to the width dimension, and the length dimension exceeding the width dimension. The detector module 130 is rotatably movable with respect to the gantry 110 for adjusting the field of view of the detector module with respect to the axis of rotation 150. The detector module is rotatably movable with respect to the gantry 110 between a first orientation 190₁ wherein the length dimension LD of the second detector array 170 extends along the axis of rotation 150, and a second orientation 190₂ wherein the length dimension of the second detector array 170 extends transversely with respect to the axis of rotation 150.

In the example illustrated in Fig. 3, the detector module is rotatably movable with respect to the gantry 110 through a rotational angle f. A magnitude of the rotational angle f separating the first orientation 190₁ from the second orientation 190₂ may be approximately 90 degrees. The rotation may be facilitated using various mechanical mechanisms, including for example by coupling the detector module 130 to the gantry via an axle. The detector module may be provided with a locking mechanism, such as a locking pin, a clamp, or a stop, and which mechanically fixes the detector module 130 at each of the first orientation 190₁ and the second orientation 190₂. The rotation may be achieved using one or more actuators, such as a motor, and which is configured to move the detector module between the first orientation 190₁ and the second orientation 190₂. X-ray attenuation data and/or gamma data may be acquired with the detector module 130 in the first orientation 190₁, and likewise with the detector module 130 in the second orientation 190₂.

The use of a detector module in which the second detector array has such length and width dimensions, or such an "aspect ratio" defined by the ratio of its length dimension LD to its width dimension WD, permits the weight of the detector module 130 to be reduced. The ability to rotate the detector module in accordance with this aspect permits the acquisition of gamma data for 2D scintigraphy images, and likewise X-ray attenuation data for X-ray projection images, with an aspect ratio that is appropriate for the imaged region of interest. For instance, data for the heart may be acquired with the length dimension LD of the second detector array 170 extending transversely with respect to the axis of rotation 150, i.e. with the detector module 130 positioned in the second orientation 190₂. By contrast, for lung imaging, or for kidney imaging, the detector module 130 may be positioned in the first orientation 190₁, i.e. with the length dimension LD of the second detector array 170 extending along the axis of rotation 150. Similarly, when X-ray attenuation data and/or gamma data is acquired from multiple angular positions q around the axis of rotation 150 by rotating the detector module 130 around the axis of rotation 130, the shape of the imaging region from which data is acquired can be controlled by positioning the detector module in the first orientation 190₁, or in the second orientation 190₂. By providing the imaging region with a shape that is appropriate for the region of interest, truncation artifacts in SPECT images that are reconstructed from the gamma data may be reduced. Such truncation artifacts result from detected gamma radiation that is emitted from regions that are outside the imaging region, or from regions that are not sampled over a complete range of angular positions around the axis of rotation.

Examples of the imaging region of the imaging system 100 for X-ray attenuation data with the detector module in each of the first orientation 190₁, and the second orientation 190₂, are illustrated respectively as imaging regions 140 and 140' in Fig. 3. As illustrated in Fig. 3, the imaging region 140' obtained with the detector module 130 in the second orientation 190₂ has a relatively larger diameter and a relatively shorter length, as compared to the imaging region 140 obtained with the detector module 130 in the first orientation 190₁.

In some examples, the detector module 130 is translatably movable with respect to the gantry 110 for adjusting the field of view of the detector module with respect to the axis of rotation 150. The detector module is translatably movable with respect to the gantry 110 in a transaxial direction with respect to the axis of rotation 150 between a first transaxial position 210₁ wherein a line 220 extending normally from the midpoint of the second detector array 170 lies on one side of the axis of rotation 150, and a second transaxial position 210₂ wherein the line 220' extending normally from the midpoint of the second detector array 170 lies on the opposite side of the axis of rotation 150.

In one example, the detector module 130 is translatably movable along a linear path 230 with respect to the gantry 110 in the transaxial direction with respect to the axis of rotation 150 between the first transaxial position 210₁ and the second transaxial position 210₂. In another example, the detector module 130 is translatably movable along a curved path 240 with respect to the gantry 110 in the transaxial direction with respect to the axis of rotation 150 between the first transaxial position 210₁ and the second transaxial position 210₂.

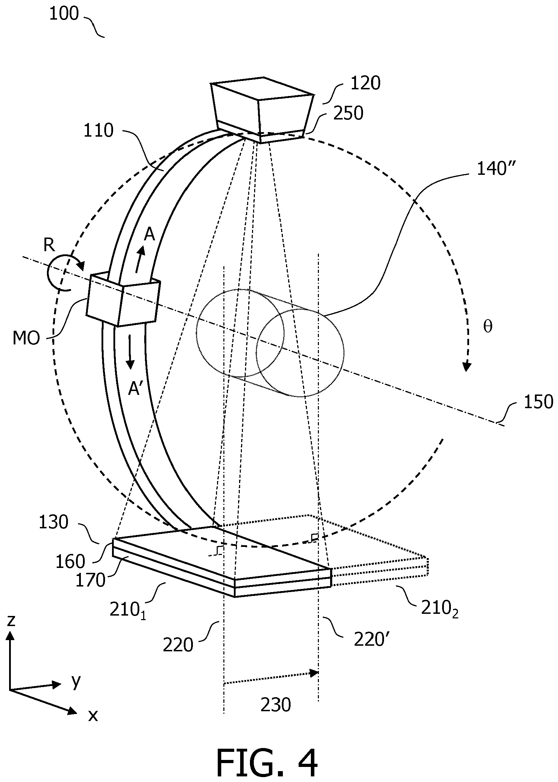

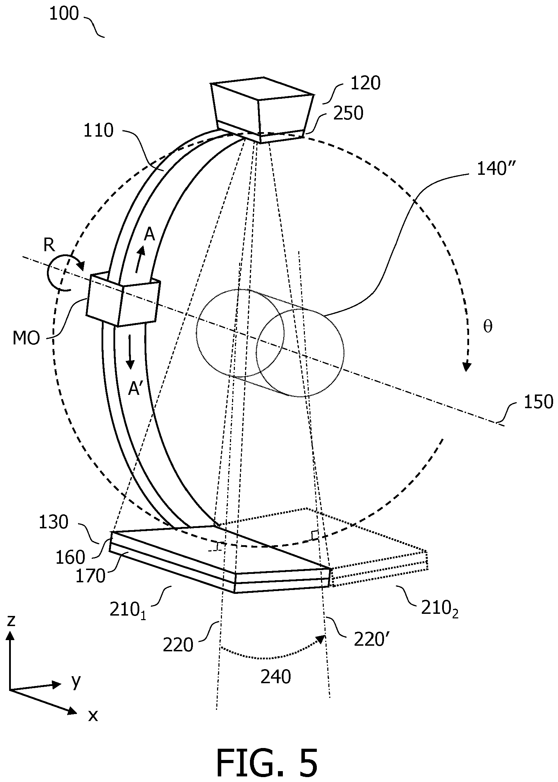

These examples are described with reference to Fig. 4, and Fig. 5. Fig. 4 is a schematic diagram illustrating a fourth example of an imaging system 100 including a detector module 130 that is coupled to a gantry 110 and wherein the detector module is movable with respect to the gantry, in accordance with some aspects of the present disclosure. Fig. 4 includes some of the features described above with reference to Fig. 1. It is noted that features illustrated in Fig. 4 that are also illustrated in Fig. 1 provide corresponding functionality, and that their functionality is not duplicated here for the sake of brevity. In the example illustrated in Fig. 4, the detector module is translatably movable along a linear path 230 with respect to the gantry 110.

In the example illustrated in Fig. 4, the second detector array 170 has a midpoint, and a line 220 is illustrated that extends normally from the midpoint. When the detector module 130 is in the first transaxial position 210₁, the line 220 lies on one side of the axis of rotation 150, and when the detector module 130 is in the second transaxial position 210₂, the line 220 lies on the other side of the axis of rotation 150. In one example, the line has a first displacement from the axis of rotation 150 when the detector module 130 is in the first transaxial position 210₁, and a second displacement from the axis of rotation 150 when the detector module 130 is in the second transaxial position 210₂, and the magnitude of the first displacement is equal to the magnitude of the second displacement. The first transaxial position 210₁, and the second transaxial position 210₂ illustrated in Fig. 4 contrast with the position illustrated in Fig. 3, and in which a line 220 extending normally from the midpoint of the second detector array 170 passes through the axis of rotation 150.

The translation between the first transaxial position 210₁, and the second transaxial position 210₂ in accordance with this example may be facilitated using various mechanical mechanisms, including for example by translating the detector module, or a guide member attached thereto, within a linear groove or a linear slot, or along a straight shaft, or by using a rack and pinion arrangement. The translation may be achieved manually, or by using one or more actuators, such as a motor, and which is configured to move the detector module between the first transaxial position 210₁, and the second transaxial position 210₂.

The ability to translate the detector module in accordance with this example permits the acquisition of gamma data, and likewise X-ray attenuation data, from an imaging region that is appropriate for the imaged region of interest. For instance, when data is acquired with the detector module 130 static and in a single angular position around the axis of rotation, the data may be acquired from imaging regions that have different offsets with respect to the axis of rotation. This may be beneficial for generating data for organs such as the heart, and individual lungs, and which are offset with respect to the axis of rotation 150. Moreover, when gamma data for a SPECT image is acquired by rotating the detector module 130 around the axis of rotation, the ability to move the detector module 130 between the first transaxial position 210₁ and the second transaxial position 210₂ permits the acquisition of a complete set of gamma data for an imaging region 140" that is extended transaxially as compared to the imaging region 140 provided by a detector module 130 in which a line 220 extending normally from the midpoint of the second detector array 170 passes through the axis of rotation 150. This can be seen by comparing the imaging region 140" in Fig. 4, with the imaging region 140 in Fig. 1. As compared to the detector module 130 in Fig. 1 in which a line extending normally from the midpoint of the second detector array 170 passes through the axis of rotation 150, the detector module 130 that is offset, as in Fig. 4, such that a line 220 extending normally from the midpoint of the second detector array 170 lies on one side of the axis of rotation 150, permits image data to be acquired from a swept volume, i.e. an imaging region, that is transaxially-extended, i.e. extended in a transaxial direction with respect to the axis of rotation 150. This principle is employed in so-called "offset geometry" cone beam CT imaging systems. However, the acquisition of gamma data for a SPECT image by rotating a detector that is disposed in such a transaxially-offset position, differs from the acquisition of X-ray attenuation data for a CT image. X-ray attenuation data is indifferent to the direction in which the X-ray radiation passes through a region of interest. This permits a full set of X-ray attenuation data to be acquired for a CT image by rotating a transaxially-offset X-ray detector through a single revolution around its axis of rotation. By contrast, the direction from which gamma radiation is received is relevant to the reconstruction of gamma data into a SPECT image. Consequently, in order to acquire a full set of gamma data for a SPECT image using a transaxially-offset detector, two rotations around the region of interest are necessary: one with the gamma detector in first transaxial position 210₁ wherein a line 220 extending normally from the midpoint of the second detector array 170 lies on one side of the axis of rotation 150, and one with the gamma detector in a second transaxial position 210₂ wherein the line 220 extending normally from the midpoint of the second detector array 170 lies on the opposite side of the axis of rotation 150. Thus, a full set of gamma data for a SPECT image with a transaxially-extended imaging region may be acquired by providing that the detector module is translatably movable between the first transaxial position and the second transaxial position.

Fig. 5 is a schematic diagram illustrating a fifth example of an imaging system 100 including a detector module 130 that is coupled to a gantry 110 and wherein the detector module is movable with respect to the gantry, in accordance with some aspects of the present disclosure. Fig. 5 includes some of the features described above with reference to Fig. 1. It is noted that features illustrated in Fig. 5 that are also illustrated in Fig. 1 provide corresponding functionality, and that their functionality is not duplicated here for the sake of brevity. In the example illustrated in Fig. 5, the detector module is translatably movable along a curved path 240 with respect to the gantry 110.

The translation between the first transaxial position 210₁, and the second transaxial position 210₂ in accordance with this example may be facilitated using various mechanical mechanisms, including for example by translating the detector module, or a guide member attached thereto, within a curved groove or a curved slot, or along a curved shaft, or by using a rack and pinion arrangement in which the rack extends along a curved path. As with the example illustrated in Fig. 4, the translation may be achieved manually, or by using one or more actuators, such as a motor, and which is configured to move the detector module between the first transaxial position 210₁, and the second transaxial position 210₂. The example illustrated in Fig. 5 offers similar advantages to the example illustrated in Fig. 4.

As mentioned above, in some examples the X-ray source 120 may also include an X-ray collimator. The X-ray collimator 250 is coupled to the X-ray source. An example of an X-ray collimator is illustrated in Fig. 1 - Fig. 5.

In one example, the X-ray collimator is movable with respect to the X-ray source for adjusting a lateral extent of X-ray radiation emitted by the X-ray source in correspondence with the adjusted field of view of the detector module 130. For instance, with reference to the example detector module 130 in Fig. 3, the X-ray collimator 250 may be configured to rotate through the same rotational angle, f, as the detector module 130. With reference to Fig. 4 and Fig. 5, the X-ray collimator may be configured to translate in the same direction as the detector module 130 such that the lateral extent of X-ray radiation emitted by the X-ray source is moved in correspondence with the adjusted field of view of the detector module 130.

In another example the X-ray collimator 250 comprises a plurality of movable plates configured to define a lateral extent of the X-ray radiation emitted by the X-ray source 120 passing through the X-ray collimator. In this example, the movable plates are configured to adjust the lateral extent of X-ray radiation emitted by the X-ray source in correspondence with the adjusted field of view of the detector module 130. In this example, the movable plates absorb X-ray radiation. The movable plates may be formed from lead, for example. The movable plates may be configured to move in a lateral direction with respect to a central beam emitted by the X-ray source, and thereby operate to limit the lateral extent of X-ray radiation emitted by the X-ray source.

By adjusting the lateral extent of X-ray radiation emitted by the X-ray source in correspondence with the adjusted field of view of the detector module 130, in accordance with these examples, it is ensured that the emitted X-ray radiation overlaps with the detector module, and that the X-ray source does not need to move with respect to the gantry. This simplifies the mechanical operation of the gantry. The collimator, and similarly movable plates, may be moved manually, or by using one or more actuators, such as a motor. For example, a motor may be used to rotate, or to translate the collimator using a worm-drive, or a rack and pinion.

In another example, the X-ray source and X-ray collimator remain in fixed positions with respect to each other, and the combined X-ray source and X-ray collimator is moved together in correspondence with the aforementioned translations of the detector module with respect to the gantry. However, this example requires a complex mechanical arrangement to move the combined X-ray source and X-ray collimator. It also requires a more robust mechanical arrangement because the X-ray source 120 can be heavy.

In some examples, the imaging system 100 described above also includes a processor 260.

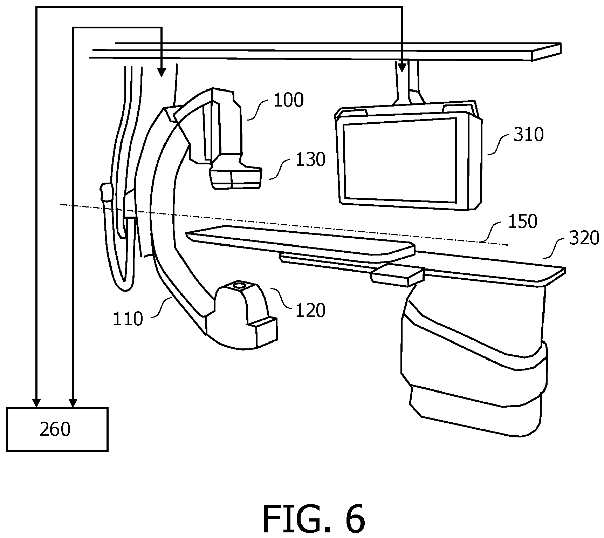

Fig. 6 is a schematic diagram illustrating an example of imaging system 100 including a processor 260, in accordance with some aspects of the present disclosure. Fig. 6 includes some of the features described above with reference to Fig. 1. It is noted that features illustrated in Fig. 6 that are also illustrated in Fig. 1 provide corresponding functionality, and that their functionality is not duplicated here for the sake of brevity. The processor 260 illustrated in Fig. 6 may generate control signals for controlling the imaging system 100. The processor 260 may alternatively or additionally receive X-ray attenuation data and/or gamma data from the imaging system 100, and generate images from the received data. The processor may perform other operations, described in more detail below. The processor 260 is in communication with the imaging system 100 in order to send and/or to receive the control signals, and likewise the data. The processor may be in communication with the imaging system 100 via any form of data communication, including wired, optical, and wireless communication. By way of some examples, when wired or optical communication is used, the communication may take place via signals transmitted on an electrical or optical cable, and when wireless communication is used, the communication may for example be via RF or optical signals.

With reference to Fig. 6, in one example, the imaging system 100 includes one or more actuators configured to rotate the gantry 110 around the axis of rotation 150; one or more actuators configured to move the detector module 130 with respect to the gantry 110 for adjusting the field of view of the detector module with respect to the axis of rotation 150; and a processor 260. The processor is configured to generate control signals for causing the one or more actuators to rotate the gantry 110 around the axis of rotation 150, and for causing the one or more actuators to move the detector module 130 with respect to the gantry.

In this example, the actuators may for example be provided by motors, as described above. By way of an example, with reference to Fig. 3, the control signals may rotate the detector module 130 with respect to the gantry 110 between the first orientation 190₁ and the second orientation 190₂. By way of another example, with reference to Fig. 4 and Fig. 5, the control signals may translate the detector module 130 between the first transaxial position 210₁ and the second transaxial position 210₂. By way of another example, with reference to Fig. 1 - Fig. 5, the control signals may rotate the gantry around the axis of rotation 150 though a predetermined range of angular positions q. For instance, the control signals may rotate the gantry around the axis of rotation 150 through an integer number of complete revolutions in a clockwise direction, or a counter-clockwise direction. By way of another example, the control signals may rotate the gantry around the axis of rotation 150 though a partial revolution in a clockwise direction, or a counter-clockwise direction. In some examples, the processor may receive feedback signals from a position sensor, such as a rotational encoder for example, and which feedback signals indicate an angular position q of the gantry around the axis of rotation 150. The processor 260 may use the control signals to rotate the gantry around the axis of rotation 150, for example to acquire data from a desired angular position q, or from a desired range of angular positions, or to provide a desired integer number of complete revolutions around the axis of rotation 150.

In one example the imaging system includes one or more actuators configured to move the X-ray collimator with respect to the X-ray source 120, or to move the movable plates, respectively. In this example, the processor 260 is configured to generate control signals for causing the one or more actuators to move the X-ray collimator with respect to the X-ray source 120, or to move the movable plates, respectively, such that an extent of the X-ray radiation emitted by the X-ray source 120 is adjusted in correspondence with the adjusted field of view of the detector module 130. In one example, the one or more actuators may move the X-ray collimator with respect to the X-ray source 120 by moving the X-ray collimator between predetermined positions. Similarly, the movable plates of the collimator may be moved to predetermined positions. The predetermined positions may be determined mechanically, or based on feedback signals from a position sensor. In the latter case, the processor 260 may receive the feedback positions and cease actuation by the one or more actuators when the predetermined position has been obtained. A position sensor such as a rotational or linear encoder may be used for this purpose, for example. In another example, the one or more actuators may move the X-ray collimator with respect to the X-ray source 120 by moving the X-ray collimator based on feedback from an X-ray detector. Similarly, the movable plates of the collimator may be moved based on feedback from an X-ray detector. In this example, the X-ray detector provides feedback to the processor 260, and the feedback represents a detected intensity of X-ray radiation from the X-ray source. The X-ray detector may be provided by one or more X-ray detector elements of the first detector array 160, or one or more separate X-ray detectors disposed in a peripheral region to the first detector array. Both examples facilitate a positioning of the collimator, or the collimator plates, in which the X-ray radiation emitted by the X-ray source 120 is limited to the detector module 130, or more specifically to the first detector array 160, or a portion thereof.

In another example, the processor 260 is configured to:
generate control signals for causing the one or more actuators to rotate the gantry 110 around the axis of rotation 150 whilst generating gamma data with the detector module 130, and with the detector module in the first transaxial position;
generate control signals for causing the one or more actuators to move the detector module 130 with respect to the gantry 110 to the second transaxial position; and
generate control signals for causing the one or more actuators to rotate the gantry 110 around the axis of rotation 150 whilst generating gamma data with the detector module 130, and with the detector module in the second transaxial position.

As mentioned above with reference to Fig. 4 and Fig. 5, the ability to move the detector module between the first transaxial position 210₁ and the second transaxial position 210₂ facilitates the acquisition of gamma data for a SPECT image, i.e. a volumetric image, with a transaxially-extended imaging region. In order to acquire a full set of gamma data for a SPECT image using a transaxially-offset detector, two rotations around the region of interest are necessary: one with the gamma detector in first transaxial position 210₁ wherein a line 220 extending normally from the midpoint of the second detector array 170 lies on one side of the axis of rotation 150, and one with the gamma detector in a second transaxial position 210₂ wherein the line 220 extending normally from the midpoint of the second detector array 170 lies on the opposite side of the axis of rotation 150. Thus, a full set of gamma data for a SPECT image with a transaxially-extended imaging region may be acquired by providing that the detector module is translatably movable between the first transaxial position and the second transaxial position.

In this example, the control signals generated by the processor 260 facilitate the acquisition of a full set of gamma data for a SPECT image. As mentioned above, the gantry 110 may be rotated around the axis of rotation 150 in various ways. For example the detector module 130 may be rotated continuously, or in a stepped manner between multiple discrete angular positions q. Thus, in one example, the control signals for causing the one or more actuators to rotate the gantry 110 around the axis of rotation 150 whilst generating gamma data, cause the one or more actuators to:
rotate the gantry 110 around the axis of rotation 150 continuously whilst generating the gamma data; or
rotate the gantry 110 around the axis of rotation 150 between a plurality of discrete angular positions q, and wherein the gamma data is generated with the gantry 110 stationary in the discrete angular positions q.

In a related example, the control signals for causing the one or more actuators to rotate the gantry 110 around the axis of rotation 150 whilst generating gamma data, are further configured to cause the one or more actuators to adjust a speed of the continuous rotation of the gantry 110 around the axis of rotation 150, or to adjust a duration in which the gantry 110 is stationary in the discrete angular positions q; and
wherein the speed of the continuous rotation of the gantry 110, or the duration in which the gantry 110 is stationary, respectively, is adjusted based on an amount of gamma radiation detected at one or more positions across the detector module 130.

In this example, the amount of gamma radiation detected at one or more positions across the detector module 130 may be measured by monitoring the gamma radiation detected by one or more detector elements of the second detector array 170. For example, one or more detector elements may be assigned, manually or automatically, to the monitoring of the gamma radiation. The detector elements may coincide with a region of interest such as the heart, or bone, for example. By monitoring the amount of radiation at the one or more positions across the detector module 130, and by adjusting the speed of the continuous rotation of the gantry 110, or the duration in which the gantry 110 is stationary, it may be assured that a desired amount of gamma radiation is detected from a specified region of interest. Similarly, the amount of gamma radiation may be monitored in two different positions: one position corresponding to a region such as the heart and in which a significant amount of radiotracer uptake is expected, and another position, corresponding to a region such as bone and in which negligible radiotracer uptake is expected. A signal to noise ratio may be calculated as the ratio between the amounts of gamma radiation detected at these two positions, and this signal to noise ratio used to adjust the speed of the continuous rotation of the gantry 110, or the duration in which the gantry 110 is stationary.

In so doing, gamma data may be acquired for a SPECT image that has a desired level of image quality.

It is noted that in the above examples in which gamma data is acquired by rotating the gantry 110 around the axis of rotation 150, it is not essential that gamma data is acquired for a complete revolution of the gantry around the axis of rotation 150. A full set of gamma data may be acquired for a cylindrical imaging region centered on the axis of rotation by acquiring gamma data by rotating the gantry 110 through a complete revolution of the gantry around the axis of rotation 150. However, for some regions of interest, such as the heart, and which are not disposed along the centerline of the body, sufficient data for a SPECT image may be generated from a partial revolution of the gantry around the axis of rotation 150. For example, sufficient data for a SPECT image of the heart, and the liver, may be obtained from a partial revolution over an angular range of approximately 180 degrees to 210 degrees. Thus, in general, in examples in which gamma data is acquired by rotating the gantry 110 around the axis of rotation, the gantry 110 may be rotated around the axis of rotation 150 through a partial revolution, or through a complete revolution, whilst generating the gamma data. In examples in which the control signals cause the one or more actuators to rotate the gantry 110 around the axis of rotation 150 whilst generating gamma data, the control signals may cause the gantry 110 to rotate around the axis of rotation 150 through a partial revolution, or through a complete revolution, whilst generating gamma data.

Similarly, various "sparse data" reconstruction techniques are known for reconstructing CT data from X-ray attenuation data that is acquired by rotating an X-ray detector through a partial revolution around the axis of rotation. Thus, X-ray attenuation data that is acquired from the detector module 130 by rotating the detector module 130 through a partial revolution around the axis of rotation, may also be reconstructed into a CT image.

In another example, the processor 260 may generate images using the data that is acquired from the detector module. Thus, in one example, the imaging system 100 includes a processor 260, and the processor is configured to:
receive the generated X-ray attenuation data and/or the generated gamma data; and
generate, from the X-ray attenuation data, a computed tomography image, or a projection X-ray image; and/or
generate, from the gamma data, a single photon emission computed tomography, SPECT, image, or a scintigraphy image.

Various techniques are known for generating images from the X-ray attenuation data and the gamma data. For example, known image reconstruction techniques may be used to reconstruct CT images and SPECT images. When both X-ray attenuation data and gamma data are acquired, the X-ray attenuation data and the gamma data may be acquired sequentially, or during time intervals that overlap one another, which helps to improve the correspondence between the two data sets. Acquiring the data sets during time intervals that overlap one another helps to reduce discrepancies between the data sets due to deformation of the region of interest. As mentioned above, data for the projection X-ray image, and likewise for the scintigraphy image may be generated with the gantry static and in a single angular position around the axis of rotation. The projection X-ray image may be used to provide an attenuation map for use in generating the scintigraphy image. Likewise, the reconstructed CT image may be used to provide an attenuation map for use in reconstructing the SPECT image. The CT image, and the SPECT image may be generated from gamma data that is acquired by rotating the gantry around the axis of rotation 150 in various ways, including by rotating the gantry continuously, or in a stepped manner, around the axis of rotation, as described elsewhere herein. In another example, the CT image and the SPECT image are generated from X-ray attenuation data and gamma data that is acquired by rotating the gantry around the axis of rotation 150 along a helical path and acquiring the X-ray attenuation data and the gamma data from multiple angular positions q around the axis of rotation. The use of such a helical path permits a region of interest to be imaged that is longer than the extent of the detector module along the axis of rotation. The helical path may be provided by translating the patient along the axis of rotation 150, or by moving the gantry 110 along the axis of rotation 150 during acquisition of the X-ray attenuation data and gamma data.

It is noted that when gamma data is acquired, the processor 260 may also apply a scatter and attenuation correction algorithm to the generated gamma data during the reconstruction of the gamma data into the single photon emission computed tomography, SPECT, image. An example of such an algorithm is disclosed in a document by Hutton, B. F., et al., "Review and current status of SPECT scatter correction", Phys. Med. Biol. 56 (2011) R85-R112. Applying a scatter correction algorithm helps to improve the quality of the reconstructed images. In one technique, a Monte-Carlo based technique may be used to account for scatter. Scatter correction may alternatively be performed by measuring gamma events outside, above and below, the photopeak energy of the isotope used for the SPECT imaging procedure. These data are used to estimate the scatter background in different projections of the SPECT procedure. This scatter correction technique may be performed prior to the reconstruction of the gamma data into a SPECT image.

The example imaging system 100 illustrated in Fig. 6 may also include various additional features. For example, the example imaging system 100 illustrated in Fig. 6 may also include: a monitor 310 for displaying images that are generated; a patient bed 320; and a user input device configured to receive user input (not illustrated in Fig. 6), such as a keyboard, a mouse, a touchscreen, and so forth.

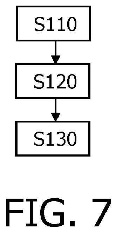

In another example, an imaging method for generating SPECT images is provided. Fig. 7 is a flowchart illustrating an example of an imaging method, in accordance with some aspects of the present disclosure. The imaging method may be used with the imaging systems 100 described above in which the detector module 130 is translatably movable with respect to the gantry 110 for adjusting the field of view of the detector module with respect to the axis of rotation 150. In other words, the imaging method may be used with the system described with reference to Fig. 4 and Fig. 5. The imaging method for generating SPECT images includes:
receiving S110, from the detector module 130, first gamma data generated by rotating the gantry 110 around the axis of rotation 150 with the detector module in the first transaxial position;
receiving S120, from the detector module 130, second gamma data generated by rotating the gantry 110 around the axis of rotation 150 with the detector module in the second transaxial position; and
reconstructing S130 the first gamma data and the second gamma data into a single photon emission computed tomography, SPECT, image.

Various SPECT image reconstruction techniques are known for this purpose. Examples of such reconstruction techniques are disclosed in a document by Zeng, G. L., "Image reconstruction - a tutorial", Computerized Medical Imaging and Graphics 25 (2001) 97 - 103. Other example techniques for SPECT image reconstruction are disclosed in a document by Bruyant, P. P., "Analytic and Iterative Reconstruction Algorithms in SPECT", Journal of Nuclear Medicine October 2002, 43 (10) 1343-1358. The first gamma data, and the second gamma data, may be acquired by rotating the gantry around the axis of rotation in a common direction, i.e. clockwise/ counter-clockwise, or by initially rotating the gantry around the axis of rotation in one direction, and subsequently rotating the gantry around the axis of rotation in the opposite direction. Rotating the gantry in the latter manner may be useful in limiting the impact of backlash during rotation. As mentioned above, it is not essential that gamma data is acquired for a complete revolution of the gantry around the axis of rotation 150. Thus, particularly when the gamma data is acquired for a partial revolution, the first gamma data may be acquired from a partial revolution in one direction, and then the direction reversed and the second gamma data may be acquired from a partial revolution in the opposite direction. By avoiding the need to perform a complete revolution, the duration of the data acquisition may be reduced, the impact of backlash may be reduced, and improved access to the subject may be provided. As mentioned above, various "sparse data" reconstruction techniques are known for reconstructing CT data from X-ray attenuation data that is acquired by rotating an X-ray detector through a partial revolution around the axis of rotation. Thus, X-ray attenuation data that is acquired from the detector module 130 by rotating the detector module 130 through a partial revolution around the axis of rotation, may also be reconstructed into a CT image.

In another example, a computer program product is provided. The computer program product may be used with the imaging systems 100 described above in which the detector module 130 is translatably movable with respect to the gantry 110 for adjusting the field of view of the detector module with respect to the axis of rotation 150. In other words, the computer program product may be used with the system described with reference to Fig. 4 and Fig. 5. The computer program product comprises instructions which when executed by one or more processors, cause the one or more processors to carry out a method of generating SPECT images, the method comprising:
receiving S110, from the detector module 130, first gamma data generated by rotating the gantry 110 around the axis of rotation 150 with the detector module in the first transaxial position;
receiving S120, from the detector module 130, second gamma data generated by rotating the gantry 110 around the axis of rotation 150 with the detector module in the second transaxial position; and
reconstructing S130 the first gamma data and the second gamma data into a single photon emission computed tomography, SPECT, image.

The above examples are to be understood as illustrative of the present disclosure, and not restrictive. Further examples are also contemplated. For instance, operations described in relation to the imaging system 100, may also be provided by the imaging method, and by the computer program product, or by the computer-readable storage medium, in a corresponding manner. It is to be understood that a feature described in relation to any one example may be used alone, or in combination with other described features, and may be used in combination with one or more features of another of the examples, or a combination of other examples. Furthermore, equivalents and modifications not described above may also be employed without departing from the scope of the invention, which is defined in the accompanying claims. In the claims, the word "comprising" does not exclude other elements or operations, and the indefinite article "a" or "an" does not exclude a plurality. The mere fact that certain features are recited in mutually different dependent claims does not indicate that a combination of these features cannot be used to advantage. Any reference signs in the claims should not be construed as limiting their scope.

## Claims

1. An imaging system (100) comprising:
a gantry (110);
an X-ray source (120); and
a detector module (130) configured to detect both X-ray radiation emitted by the X-ray source (120), and gamma radiation;
wherein the X-ray source (120) and the detector module (130) are coupled to the gantry (110) in an opposing configuration for generating both X-ray attenuation data representing an attenuation of the X-ray radiation passing through an imaging region (140) disposed between the X-ray source and the detector module, and gamma data representing gamma radiation received from the imaging region (140);
wherein the gantry (110) comprises an axis of rotation (150), and the gantry is configured to rotate around the axis of rotation for detecting the X-ray radiation and the gamma radiation with the detector module from a plurality of angular positions (q) around the axis of rotation; and
wherein the detector module (130) is movable with respect to the gantry (110) for adjusting a field of view of the detector module with respect to the axis of rotation.

2. The imaging system (100) according to claim 1, wherein the detector module (130) comprises a first detector array (160) configured to detect the X-ray radiation, and a second detector array (170) configured to detect the gamma radiation.

3. The imaging system (100) according to claim 2, wherein the first detector array (160) and the second detector array (170) comprise planar layers.

4. The imaging system (100) according to claim 3, wherein the planar layers are stacked along a path (180) extending through both the X-ray source (120) and a center of the detector module (130); and
wherein the first detector array is disposed relatively closer to the X-ray source (120) than the second detector array (170).

5. The imaging system (100) according to any one of claims 2 - 4, wherein the second detector array (170) comprises a length dimension (LD) and a width dimension (WD), the length dimension being measured orthogonally with respect to the width dimension and the length dimension exceeding the width dimension; and
wherein the detector module (130) is rotatably movable with respect to the gantry (110) for adjusting the field of view of the detector module with respect to the axis of rotation (150), the detector module being rotatably movable with respect to the gantry (110) between a first orientation (190₁) wherein the length dimension (LD) of the second detector array (170) extends along the axis of rotation (150), and a second orientation (190₂) wherein the length dimension of the second detector array (170) extends transversely with respect to the axis of rotation (150).

6. The imaging system (100) according to any one of claims 2 - 5, wherein the detector module (130) is translatably movable with respect to the gantry (110) for adjusting the field of view of the detector module with respect to the axis of rotation (150), the detector module being translatably movable with respect to the gantry (110) in a transaxial direction with respect to the axis of rotation (150) between a first transaxial position (210₁) wherein a line (220) extending normally from the midpoint of the second detector array (170) lies on one side of the axis of rotation (150), and a second transaxial position (210₂) wherein the line (220') extending normally from the midpoint of the second detector array (170) lies on the opposite side of the axis of rotation (150).

7. The imaging system (100) according to claim 6, wherein the detector module (130) is translatably movable along a linear path (230) with respect to the gantry (110) in the transaxial direction with respect to the axis of rotation (150) between the first transaxial position (210₁) and the second transaxial position (210₂); or
wherein the detector module (130) is translatably movable along a curved path (240) with respect to the gantry (110) in the transaxial direction with respect to the axis of rotation (150) between the first transaxial position (210₁) and the second transaxial position (210₂).

8. The imaging system (100) according to any one of claims 1 - 7, wherein the X-ray source (120) comprises an X-ray collimator (250);
wherein the X-ray collimator (250) is coupled to the X-ray source; and
wherein the X-ray collimator is movable with respect to the X-ray source for adjusting a lateral extent of X-ray radiation emitted by the X-ray source in correspondence with the adjusted field of view of the detector module (130);
or
wherein the X-ray collimator (250) comprises a plurality of movable plates configured to define a lateral extent of the X-ray radiation emitted by the X-ray source (120) passing through the X-ray collimator, and wherein the movable plates are configured to adjust the lateral extent of X-ray radiation emitted by the X-ray source in correspondence with the adjusted field of view of the detector module (130).

9. The imaging system (100) according to any previous claim, wherein the imaging system further comprises:
one or more actuators configured to rotate the gantry (110) around the axis of rotation (150);
one or more actuators configured to move the detector module (130) with respect to the gantry (110) for adjusting the field of view of the detector module with respect to the axis of rotation (150); and
a processor (260); and
wherein the processor is configured to generate control signals for causing the one or more actuators to rotate the gantry (110) around the axis of rotation (150), and for causing the one or more actuators to move the detector module (130) with respect to the gantry.

10. The imaging system (100) according to claim 9 when dependent on claim 8, wherein the imaging system further comprises one or more actuators configured to move the X-ray collimator with respect to the X-ray source (120), or to move the movable plates, respectively; and
wherein the processor (260) is configured to generate control signals for causing the one or more actuators to move the X-ray collimator with respect to the X-ray source (120), or to move the movable plates, respectively, such that the lateral extent of the X-ray radiation emitted by the X-ray source (120) is adjusted in correspondence with the adjusted field of view of the detector module (130).

11. The imaging system (100) according to claim 9 when dependent on claim 7 or claim 8, wherein the processor (260) is configured to:
generate control signals for causing the one or more actuators to rotate the gantry (110) around the axis of rotation (150) whilst generating gamma data with the detector module (130), and with the detector module in the first transaxial position;
generate control signals for causing the one or more actuators to move the detector module (130) with respect to the gantry (110) to the second transaxial position; and
generate control signals for causing the one or more actuators to rotate the gantry (110) around the axis of rotation (150) whilst generating gamma data with the detector module (130), and with the detector module in the second transaxial position.

12. The imaging system (100) according to claim 11, wherein the control signals for causing the one or more actuators to rotate the gantry (110) around the axis of rotation (150) whilst generating gamma data, cause the one or more actuators to:
rotate the gantry (110) around the axis of rotation (150) continuously whilst generating the gamma data; or
rotate the gantry (110) around the axis of rotation (150) between a plurality of discrete angular positions (q), and wherein the gamma data is generated with the gantry (110) stationary in the discrete angular positions (q).

13. The imaging system (100) according to claim 12, wherein the control signals for causing the one or more actuators to rotate the gantry (110) around the axis of rotation (150) whilst generating gamma data, are further configured to cause the one or more actuators to adjust a speed of the continuous rotation of the gantry (110) around the axis of rotation (150), or to adjust a duration in which the gantry (110) is stationary in the discrete angular positions (q); and
wherein the speed of the continuous rotation of the gantry (110), or the duration in which the gantry (110) is stationary, respectively, is adjusted based on an amount of gamma radiation detected at one or more positions across the detector module (130).

14. The imaging system (100) according to claim 1, further comprising a processor (260), and wherein the processor is configured to:
receive the generated X-ray attenuation data and/or the generated gamma data; and generate, from the X-ray attenuation data, a computed tomography image, or a projection X-ray image; and/or
generate, from the gamma data, a single photon emission computed tomography, SPECT, image, or a scintigraphy image.

15. An imaging method for generating SPECT images using the system according to claim 6, the method comprising:
receiving (S110), from the detector module (130), first gamma data generated by rotating the gantry (110) around the axis of rotation (150) with the detector module in the first transaxial position;
receiving (S120), from the detector module (130), second gamma data generated by rotating the gantry (110) around the axis of rotation (150) with the detector module in the second transaxial position; and
reconstructing (S130) the first gamma data and the second gamma data into a single photon emission computed tomography, SPECT, image.
